# EUROPEAN PATENT SPECIFICATION

(11) **EP 2 097 021 B1**
(45) Date of publication and mention of the grant of the patent: **13.08.2014**
(21) Application number: 07853138.1
(22) Date of filing: 20.11.2007
(51) Int. Cl.: A61B 17/34, A61B 19/00

(54) **MRI IMAGEABLE ASSEMBLY**
MRT DETEKTIERBARER OBTURATOR
ENSEMBLE D'IMAGERIE PAR IRM

(30) Priority: 24.11.2006 US 860887 P; 30.11.2006 US 872020 P; 30.10.2007 US 980302
(43) Date of publication of application: 09.09.2009
(73) Proprietor: Senorx, Inc., Tempe, AZ 85280-1740 (US)
(72) Inventor: LUBOCK, Paul, Laguna Niguel, CA 92677 (US); QUICK, Richard L., Mission Viejo, CA 92692 (US); BROADAWAY, Ethan, Huntington Beach, CA 92647 (US)
(74) Representative: Tomlinson, Edward James
(86) International application number: PCT/US2007/024249
(87) International publication number: WO 2008/063636

(56) References cited:
- EP-A- 1 114 618
- WO-A-02/41786
- WO-A-2005/013832
- WO-A-2007/069105
- WO-A-2008/016551
- FR-A- 2 853 521
- US-A1- 2003 233 101
- US-A1- 2006 241 385
- US-B1- 6 220 248
- US-B2- 6 939 318

## Description

### FIELD OF THE INVENTION

This invention relates generally to the field of medical devices. In particular, the invention relates to devices for marking a biopsy site.

### BACKGROUND OF THE INVENTION

In modern medical practice small tissue samples, known as biopsy specimens, are often removed from tumors, lesions, organs, muscles and other tissues of the body. Such removal of tissue samples may be accomplished by open surgical technique (i.e., removal of a small sample of tissue through a small surgical incision using a local anesthetic), or through the use of a specialized biopsy instrument such as a biopsy needle. After the tissue samples have been removed, they are typically subjected to diagnostic tests or examinations such as a) gross and microscopic examination to determine cytology and/or histology, b) biochemical analyses to determine the presence or absence of chemical substances which indicate certain disease states, c) microbiological culturing to determine the presence of bacteria or other microbes, and/or d) other diagnostic procedures. The information obtained from these diagnostic tests and/or examinations can then be used to make or confirm diagnoses and/or to formulate treatment plans for the patient.

When performing an image guided biopsy procedure an obturator is used as a place holder and is placed in tissue such that its distal tip will be located at the point in the patient's body where the biopsy is to be taken or where a biopsy site marker or tissue marker is to be placed after a biopsy procedure. Subsequent images are acquired that can confirm the correct placement of the obturator. When the obturator is placed at the desired location within the body, blood can enter the lumen of the obturator prior to delivery of the tissue markers. This backflow of blood into the obturator creates a risk of blood clotting.

Current obturators are constructed of homogeneous materials. During magnetic resonance imaging (MRI) guided biopsies, the tip of the obturator is located by indexing through many cross sectional views (typically every 2mm, but higher and lower discriminations are possible). The material of the obturator will be distinguishable in the cross sectional images to a varying degree depending on the morphology of the tissue and the obturator's own material makeup. Since the obturator is homogeneous, the signature of the obturator will not vary from one cross-sectional image to the next along its length. The tip of the obturator is located by selecting the first cross-sectional image in which the obturator is not seen. This result can be visually ambiguous depending on the relative strength of the image signature of the obturator compared to the surrounding tissue.

After the biopsy sample is taken, it may take several days or weeks before the results of the examination of the sample are obtained, and still longer before an appropriate treatment decision is reached. If the decision involves surgery, it is clearly important for the surgeon to find the location in the breast from where the tumor tissue has been taken in the biopsy procedure, so that the entire tumor and possibly surrounding healthy tissue can be removed.

However, radiographically imageable tissue features, originally detected in a mammogram, may be removed, altered or obscured by the biopsy procedure. In order for the surgeon or radiation oncologist to direct surgical or radiation treatment to the precise location of the breast lesion several days or weeks after the biopsy procedure was performed, it is desirable that one or more biopsy site markers be placed in or on the patient's body to serve as a landmark for subsequent location of the lesion. The purpose of such markers is to facilitate the surgical procedure that is performed while the marker is detectable. US 6,939,318 B2 describes a system for deploying an implant into a body comprising an inner housing having piercing jaws, an outer housing also with piercing jaws and an inner plunger assembly.

The present invention provides a marker delivery device for placing an obturator at the desired site in a patient's body as a placeholder and for delivering such markers into the biopsy cavity.

### SUMMARY OF THE INVENTION

The present invention provides an MRI imageable marker delivery assembly according to claim 1. Further preferred aspects of the invention are provided according to the dependent claims. This invention relates to devices for placement of an intracorporeal object that functions as a marker, a therapeutic agent or a diagnostic agent and particularly for placing an obturator at a desired location within a patient's body and for delivering one or more intracorporeal objects through the obturator to that location. The obturator may operate as a place-holder during an image guided procedures such as a biopsy. The distal end of the obturator is placed where the procedure is to be performed or one or more intracorporeal objects or bodies are to be delivered.

In one embodiment having features of the present invention the device includes an obturator which has an elongated shaft with a internal lumen, a proximal end, and a substantially sealed distal end which prevents or minimizes the backflow of body fluids, such as blood, though the lumen of the obturator. The substantially sealed distal end can be a penetratable membrane or may have petals or a duckbill-type valve which are configured to allow passage of one or more intracorporeal objects or a delivery tube with one or more intracorporeal objects therethrough while preventing or minimizing entry of body fluids into the inner lumen of the obturator. Preferably the obturator is configured to fit within a procedure cannula, e.g. a cannula of a biopsy device, for example, the cannula of SenoRx's EnCor™ Magnetic Resonance Imaging Breast Biopsy System. The cannula provides access to the desired location within the patient's body.

The delivery tube has a delivery lumen configured to contain one or more intracorporeal objects. The distal tip is configured to penetrate the substantially sealed distal end of the obturator so that the intracorporeal bodies can be delivered while the obturator is in place within the body. The shape of the distal tip may be sharp or needle like when the sealed distal end of the obturator has a membrane or it may be blunt or rounded when the distal end of the obturator is petalled or has a one-way valve.

The device preferably further includes a plunger having an elongated shaft with a proximal portion and a distal portion. The plunger is configured to be slidably disposed within the lumen of the delivery tube and is located proximal to the one or more intracorporeal objects within the lumen thereof. When the plunger is extended distally within the lumen, the distal end thereof moves one or more intracorporeal objects toward and eventually through the distal end of the delivery tube. The plunger preferably has an enlarged proximal end to prevent the distal portion of the plunger from advancing too far within the delivery lumen. Alternatively, a fluid maybe used to advance the intracorporeal objects through the opening.

In use, the obturator is placed at a desired location within a patient's body. The delivery tube is advanced distally within the obturator until the distal tip passes through the substantially sealed distal end of the obturator. Next the plunger is advanced distally within the delivery tube so that at least one intracorporeal object is pushed though the opening of the distal tip.

In one embodiment of the device the distal portion of the obturator includes a detectable element capable of producing a significant image signature at the location in the patient's body where the distal portion of the obturator is placed. Preferably this embodiment includes an obturator having an elongated shaft, a proximal end, a substantially sealed distal end, and a detectable element, preferably in the form of a ring at or near the distal end. A detectable element capable of producing a significant image signature is located adjacent to the substantially sealed distal end, preferably in the form of ring at the junction between the distal tip and the elongated shaft.

The devices and systems of the present invention offer improved delivery by minimizing the backflow of body fluids, such as blood, though the obturator lumen and thereby decreasing a risk of clot formation in the obturator. These and other advantages of the invention will become more apparent from the following detailed description when taken in conjunction with the accompanying drawings.

### BRIEF DESCRIPTION OF THE DRAWINGS

Figure 1A is **an** elevational view of an assembly having features of the invention including a marker delivery shaft and an obturator.
Figure 1B is a transverse cross sectional view of the obturator of Figure 1A taken along line 1B-1B.
Figure 1C is a transverse cross sectional view of the marker delivery shaft of Figure 1A taken along lines 1C-1C.
Figures 2A and 2B are elevational views of a delivery device embodying features of the invention wherein the distal tip of the marker delivery shaft is proximal to the substantially sealed distal end. Figure 2B is rotated 90° with respect to Figure 2A.
Figure 2C is a longitudinal cross sectional view taken along lines 2C-2C in Figure 2A.
Figure 2D is a transverse cross sectional view taken along lines 2D-2D in Figure 2A.
Figure 3 is a perspective view of a substantially sealed distal end of an obturator having one or more petals.
Figure 4 is a perspective view of a substantially sealed distal end of an obturator having a duck billed valve.
Figure 5 is an elevational view a distal tip of a marker delivery tubular shaft having a needle-like shape.
Figure 6 is an elevational view of a distal tip of a marker delivery tubular shaft having a blunt end.
Figures 7A and 7B are elevational views of a proximal portion of an obturator embodying features of the invention wherein the distal tip of the marker delivery shaft has partially punctured the substantially sealed distal end of the obturator and the plunger is not yet deployed. Figure 7B is rotated 90° with respect to Figure 7A.
Figure 7C is a longitudinal cross sectional view taken along line 7C-7C in Figure 7A.
Figures 8A and 8B are elevational views of a proximal portion of an obturator embodying features of the invention wherein the distal tip of the marker delivery shaft has completely punctured the substantially sealed distal end of the obturator and the plunger is not yet deployed. Figure 8B is rotated 90° with respect to Figure 8A.
Figure 8C is a longitudinal cross sectional view taken along lines 8C-8C in Figure 8A.
Figures 9A and 9B are elevational views of a proximal portion of an obturator embodying features of the invention wherein the distal tip of the marker delivery shaft has partially punctured the substantially sealed distal end of the obturator and the plunger is not yet deployed. Figure 9B is rotated 90° with respect to Figure 9A.
Figure 9C is a longitudinal cross sectional view taken along line 9C-9C in Figure 9A.
Figure 10A is an elevational view of an obturator embodying features of the invention including a detectable element.
Figure 10B is a cross sectional view of the obturator shown in Figure 10A taken along line 10B-10B.
Figure 10C is an enlarged view of Section C in Figure 10A.
Figure 10D is a longitudinal cross sectional view of the distal end of the obturator taken along line 10D-10D in Figure 10C.
Figure 10E is an enlarged view of section E shown in Figure 10D.
Figure 10F is an elevational view of the obturator wherein the distal end of the obturator, the detectable element, and the proximal portion of the obturator are shown separated.
Figure 10G is an enlarged view of Section G shown in Figure 10F.
Figure 11 A is an elevational view of an obturator embodying features of the invention including a plurality of detectable elements or bodies within the obturator.
Figure 11 B is a longitudinal cross-sectional view of the obturator shown in Figure 11A taken along line 11 B-11 B.
Figure 11C is a longitudinal cross-sectional view of the obturator as in Figure 11 B with the plunger distally advanced to discharge the detectable elements or bodies within the obturator.

### DETAILED DESCRIPTION OF THE INVENTION

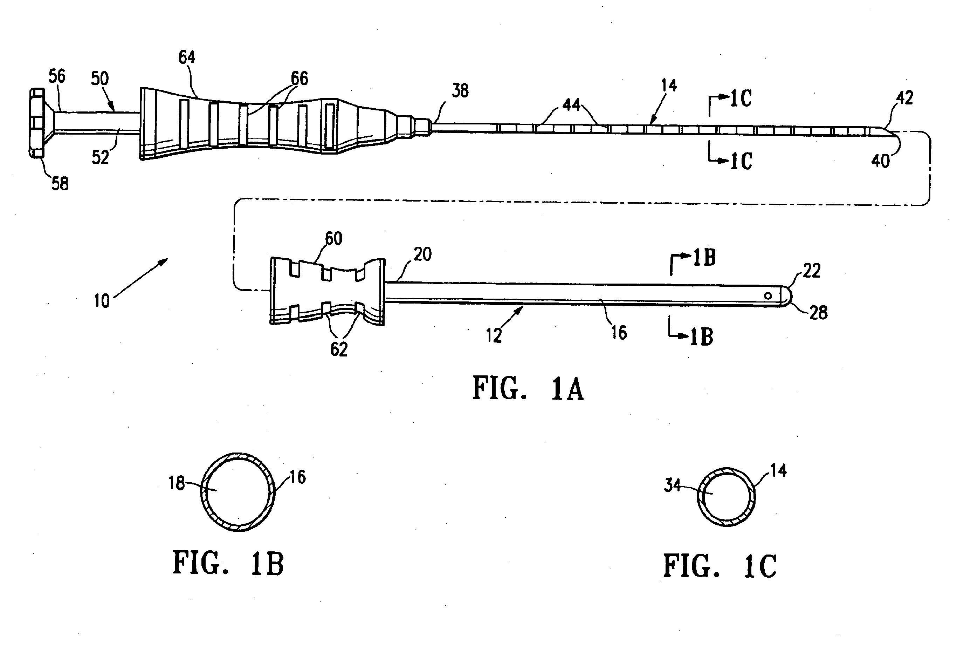

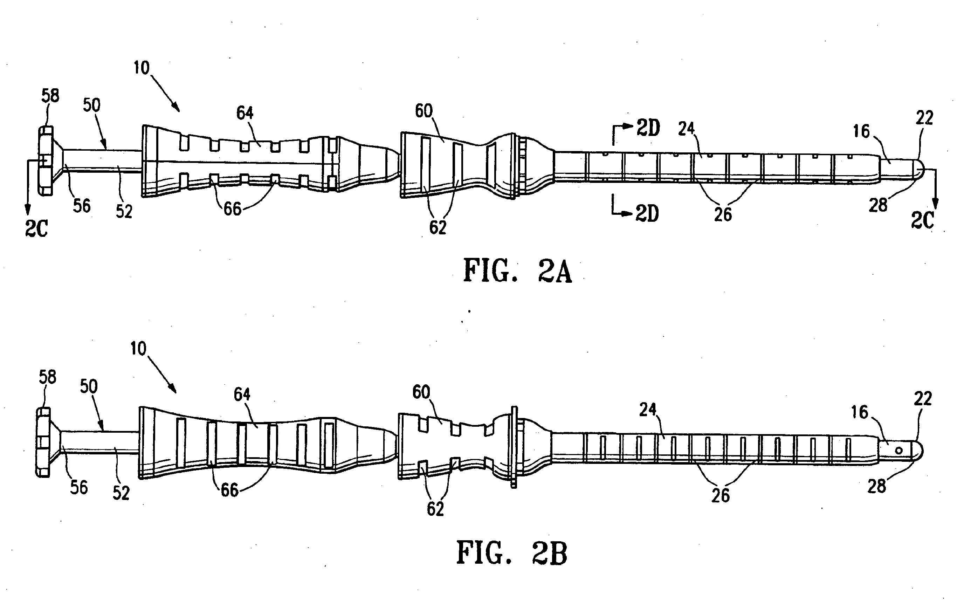

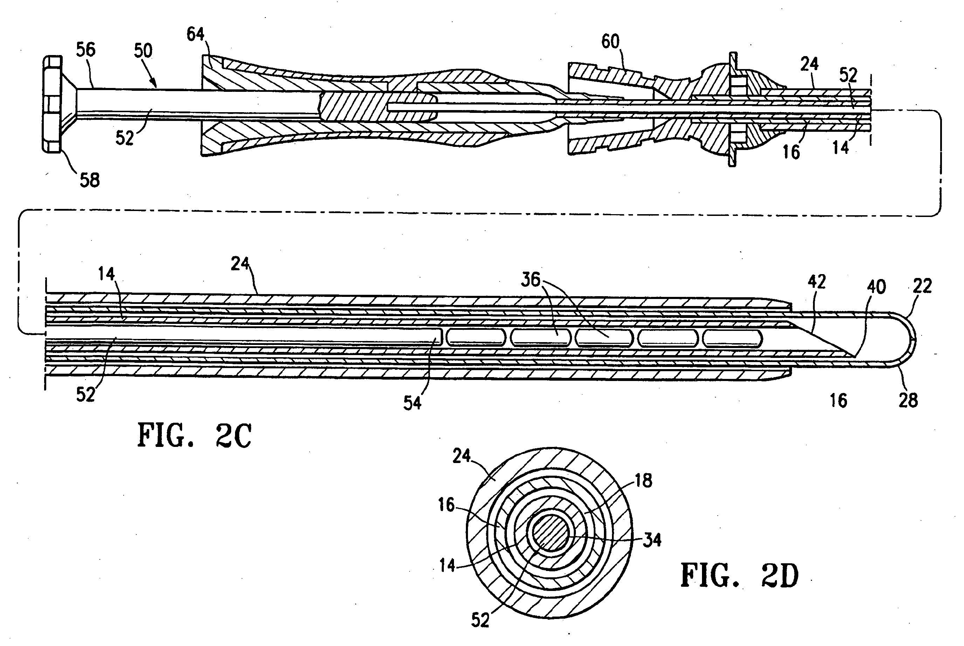

Figures 1A-2D shows an embodiment of a marker delivery device 10 having features of the invention including an obturator 12 and a marker delivery tubular shaft 14. The obturator 12 has an elongated shaft 16, an internal lumen 18, a proximal end 20 and a substantially sealed distal end 22. Preferably, as shown in Figures 2A-2D, the obturator 12 is configured to fit within a cannula 24 of a biopsy device, such as SenoRx's EnCor™ Magnetic Resonance Imaging (MRI) Breast Biopsy System. The cannula 24 provides access to the desired location within a patient's body. In some embodiments the cannula 24 includes depth markings 26 which indicate the distance which the obturator 12 has advanced within the patient's body.

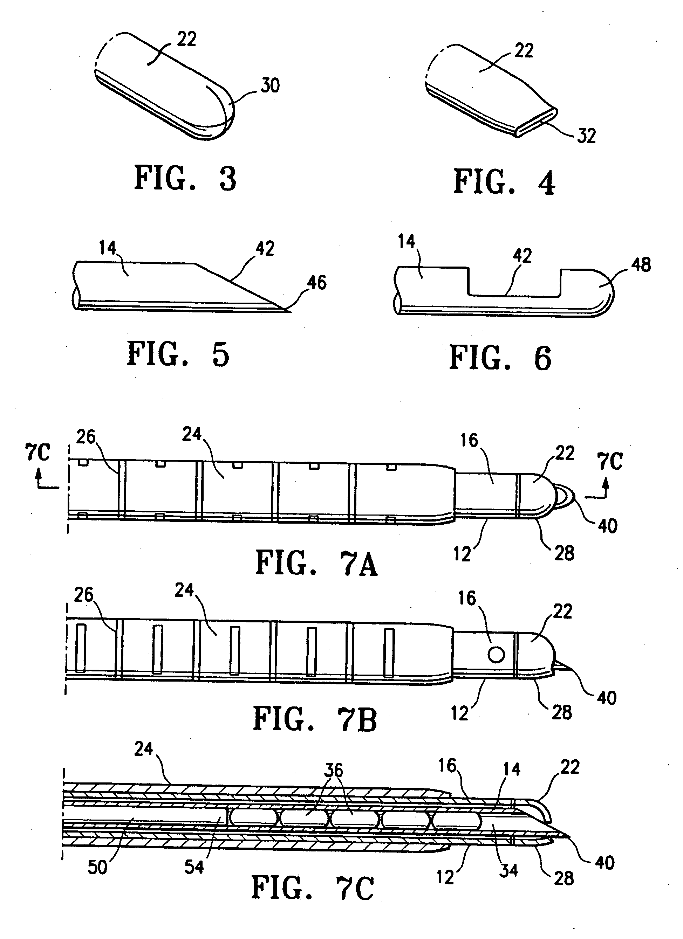

The substantially sealed distal end 22 of the obturator 12 is configured to prevent or minimize the backflow of fluids, such as body fluids, through the internal lumen 18 of the obturator 12. Preferably the substantially sealed distal end 22 is formed of a penetratable membrane 28. Alternatively the substantially sealed distal end 22 is formed of two or more petals 30 (Figure 3) or can be formed of a duck-billed valve 32 (Figure 4).

The marker delivery tubular shaft 14 is configured to be slidably disposed within the internal lumen 18 of the obturator 12. The tubular shaft 16 has a marker delivery lumen 34 configured to contain one or more tissue markers 36 for marking a biopsy site, a proximal end 38, and a distal tip 40 with an opening 42 for passage of one or more of the markers 36. The tissue markers 36 may be those described in U.S. Patent No. 6,996,433, U.S. Patent No. 6,993,375, U.S. Patent No. 6,862,470, U.S. Patent No. 6,725,083, U.S. Patent No. 6,662,041, U.S. Patent No. 6,567,689, U.S. Patent No. 6,427,081, 6,347,241, U.S. Patent No. 6,161,034, U.S. Patent Application No. 10/444,770, U.S. Patent Application No. 10/444,428, and U.S. Patent Application No. 10/001,043. The marker delivery tubular shaft 14 preferably also includes depth markings 44 which indicate the distance which the tubular shaft 14 has advanced within the obturator 12.

The distal tip 40 of the marker delivery shaft 14 is configured to penetrate the substantially sealed distal end 22 of the obturator 12 so that tissue markers 36 can be delivered while the obturator 12 is in place within the patient's body. Preferably the distal tip 40 is needle shaped 46 (Figure 5), however, the distal tip can alternatively be a blunt tip 48 (Figure 6) which is capable of penetrating a substantially sealed distal end 22 that is formed of a penetratable membrane 28 which is weakened or a distal end 22 with petals 30 or a valve 32.

Preferably the marker delivery device 10 also includes a plunger 50 having an elongated shaft 52 with a distal end 54 and a proximal end 56. The plunger 50 is configured to be slidably disposed within the marker delivery lumen 34 and is located proximal to the one or more tissue markers 36 within the marker delivery lumen 34. When the plunger 50 is extended distally within the marker delivery lumen 34 it moves one or more tissue markers toward and eventually through the opening 42 in the distal tip 40 of the marker delivery shaft 14. The plunger 50 preferably has an enlarged proximal end 58 to prevent its entry into the lumen 34. Alternatively, a fluid (not shown) may be used to advance the markers 36 through the opening 42 in the distal tip 40 of the marker delivery tubular shaft 14.

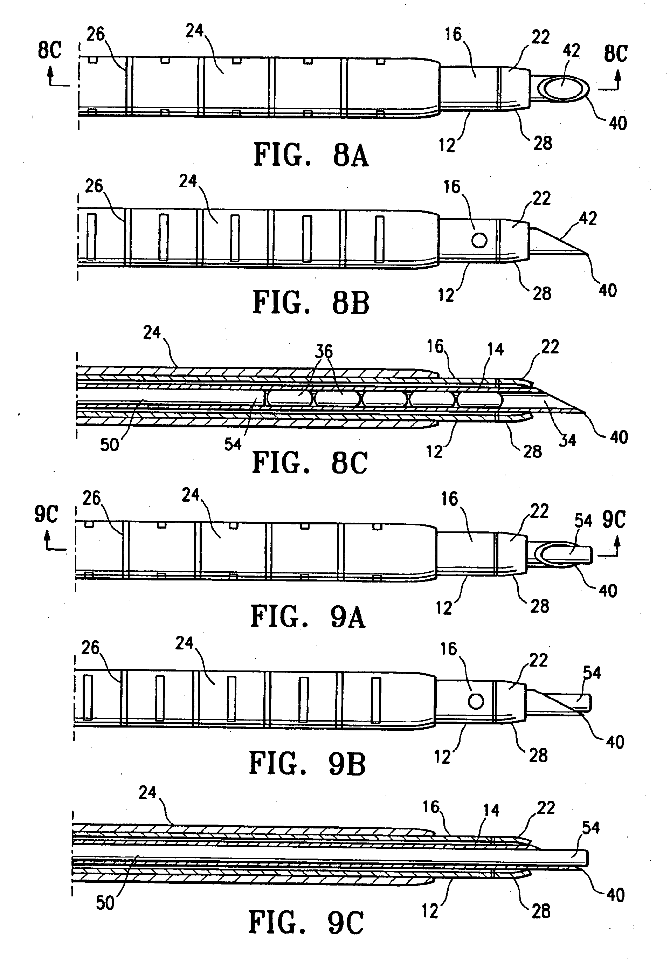

Figures 7A-7C show the distal tip 40 of the marker delivery tube 14 partially penetrating the substantially sealed distal end 22 of the obturator 12. The tissue markers 36 in Figure 7A-7C are contained within the marker delivery lumen 34. Figures 8A-8C show the distal tip 40 completely penetrating the substantially sealed distal end 22 of the obturator 12 and the tissue markers 36 within the marker delivery lumen 34. Figures 9A-9C show the distal tip 40 of the marker delivery tubular shaft 14 completely penetrating the substantially sealed distal end 22. In Figures 9A-9C the plunger 50 is deployed distally within the marker delivery lumen 34.

Preferably the obturator 12 has a hub 60 at the proximal end 20 of the obturator shaft 16. The hub 60 may have gripping ridges 62 which allow a person operating the device 10 to maintain a grip on the device 10. The marker delivery tubular shaft 14 preferably also has a hub 64 at the proximal end of the marker delivery shaft 38 with gripping ridges 66. At least a portion of the hub 64 of the marker delivery tubular shaft 14 is configured to fit within the hub 64 of the obturator 12 when the marker delivery shaft 14 is inserted into the obturator 12.

The marker delivery device 10 is preferably formed of a non-magnetic material. A plastic such as MAKROLON®, a polycarbonate from Bayer Material Sciences a division of Bayer AG, is suitable and will not interfere with a magnetic resonance imaging device (MRI). The device may also include a radiopaque material which allows for detection of the device. Alternatively the location of the obturator 12 may be determined by detecting air within the elongated shaft 16 of the obturator 12 with a magnetic resonance imaging device (not shown).

In use, the obturator 12 of the device 10 is inserted into a desired location within a patient's body. Preferably the obturator 12 is placed within the cannula 24 of a biopsy device that remains in the patient's body after the biopsy device is removed. The marker delivery tubular shaft 14 is then inserted into the obturator 12 and the distal tip 40 of the tubular shaft 14 is advanced through the substantially sealed distal end 22 of the obturator 12. At least one tissue marker 36 within the marker delivery tubular shaft 14 is then advanced distally through the opening 42 in the distal tip 40 of the marker delivery tubular shaft 14. Preferably a plunger 50 is advanced distally within the tubular shaft 14 so that at least one tissue marker 36 is moved through the opening 42 in the distal tip 40. Alternatively a fluid (not shown) can be used in place of the plunger to move the tissue markers 36 through the distal tip 40.

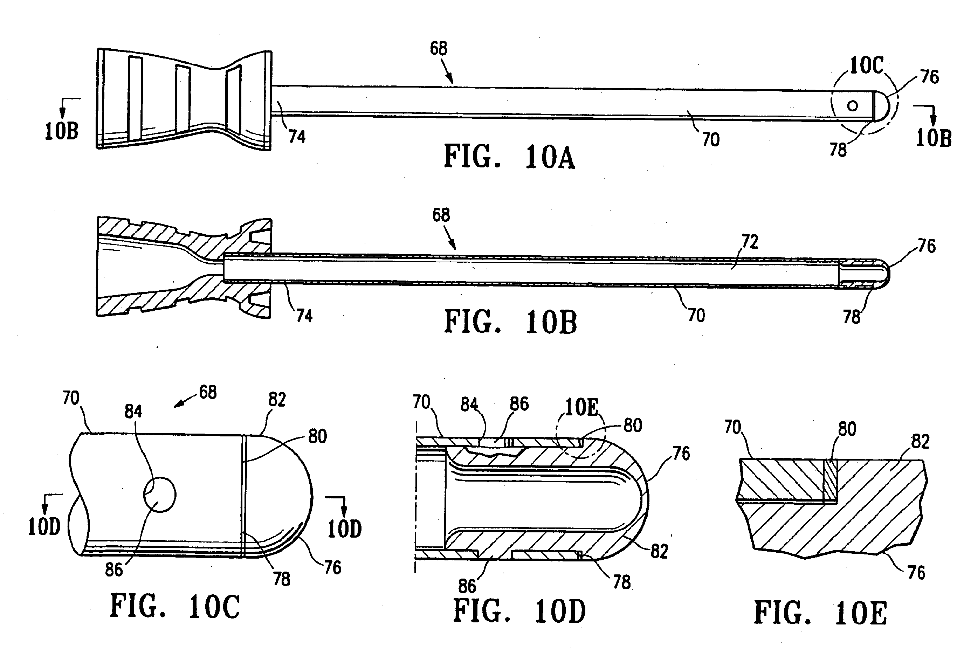

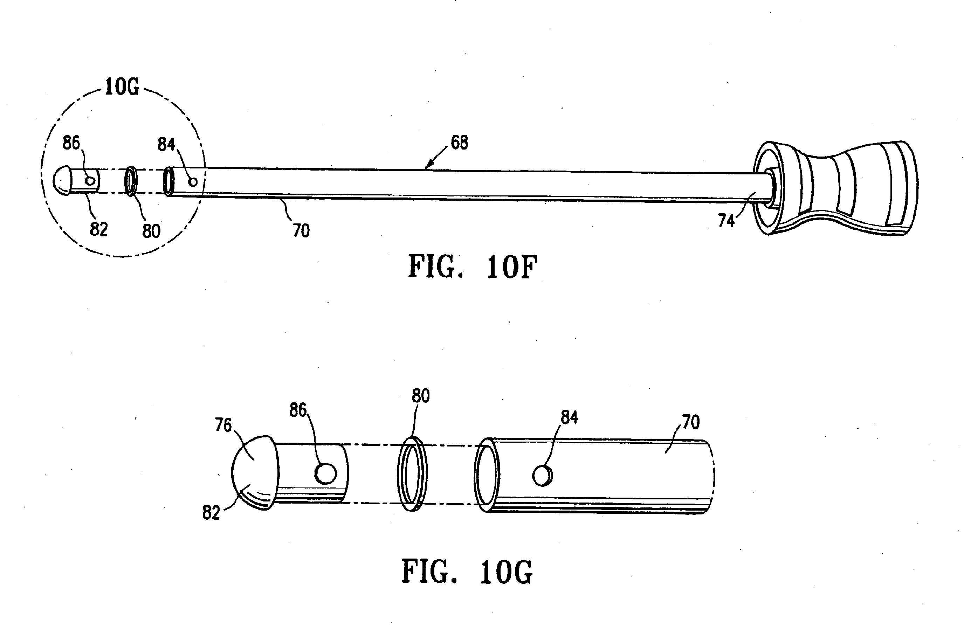

An embodiment of the device having features of the invention, which is shown in Figures 10A-10G, includes an obturator 68 having an elongated shaft 70 with an internal lumen 72, a proximal end 74, a substantially sealed distal end 76, and a detectable element 78 capable of producing a relatively significant image signature. Preferably the detectable element 78 is incorporated into the obturator 68 at a location determined to be optimum for complimenting the subsequent procedure.

As shown in Figure 10G, the detectable element 78 preferably is in the form of a ring 80 located near the substantially sealed distal end 76 of the obturator 68. Preferably the substantially sealed distal end 76 of the obturator 68 is located on an obturator tip 82. The obturator tip 82 also includes at least one aperture 84 and the elongated shaft 70 of the obturator 68 preferably includes a raised tab 86 which extends into the aperture 84 to hold the obturator tip 82 in place. The detectable element 78 in ring form 80 may be placed at the point where the obturator tip 82 and the elongated shaft 70 of the obturator 68 meet.

Preferably the detectable element 78 is in a form which allows the internal lumen of the obturator 72 to remain unobstructed, such as a ring 80. The detectable element 78 can also be a small sphere or rod, one or more wires, or a collar. Also any plan-form shape constructed of sheet material, either planar or formed into a non-planar shape. Alternatively, the detectable element 78 may be the entire tip 82 of the obturator 68. Alternatively more than one detectable element 78 could be incorporated into the obturator 68 to be used as a marker capable of determining the depth of the obturator 68 within a patient's body (not shown).

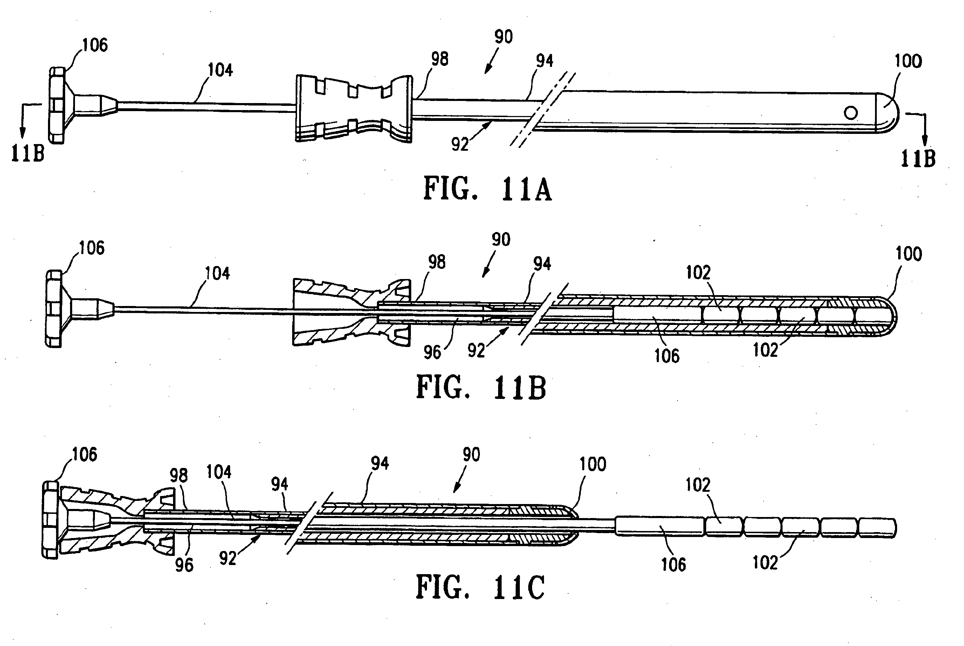

Figures 11A-C illustrate an alternative embodiment of an object delivery device 90 having features of the invention including an obturator 92 which has an elongated shaft 94, an internal lumen 96, a proximal end 98 and a substantially sealed distal end 100. Preferably, as previously discussed with the other embodiments, the obturator 92 is configured to fit within a cannula of a biopsy device, such as SenoRx's EnCor™ Magnetic Resonance Imaging (MRI) Breast Biopsy System. The substantially sealed distal end 100 of the obturator 92 is configured to prevent or minimize the backflow of fluids, such as body fluids, through the inner lumen 96. A plurality of intracorporeal objects 102, e.g. biopsy site markers, are disposed within the inner lumen 96. A plunger 104 with an enlarged head 106 is slidably disposed in part within the inner lumen 96 proximal to the plurality of intracorporeal objects 102. Distal movement of the plunger 104 pushes the objects 102 through the distal end into a body site. In this embodiment, the intracorporeal objects may not be able to penetrate a membrane, so the substantially sealed distal end 100 may be formed of two or more petals such as shown in Figure 3 or can be formed of a duck-billed valve such as shown in Figure 4.

Suitable materials for use as the detectable element 78 are metal, ceramic, metal filled plastic, mineral filled plastic, or a radiopaque material. Radiopaque materials such as stainless steel, platinum, gold, iridum, tantalum, tungsten, silver, rhodium, nickel, bismuth or other radiopaque metals, mixtures of radiopaque metals, oxides of radiopaque metals, barium salts, iodine salts, iodinated materials, and combinations thereof are suitable as well. Additionally, MRI contrast agents such as Gadolinium and vitamin E may also be employed.

If desired, an imageable stylet may be used within the inner lumen of the sealed obturator to show the axis of the instrument and the depth of the insertion within the body. Preferably the stylet is formed of material which is compatible with MRI and which is seen in MRI generated images. Suitable materials include non-magnetic metals, non-magnetic metal filled plastics, hollow tubes filled at least in part with an MRI visible substance such as Gadolinium or other fluids. See for example the attached disclosure entitled MRI Visible Obturator. As indicated in the disclosure, the obturator may also or in lieu of have a length formed of MRI imageable material.

While particular forms of the invention have been illustrated and described herein directed to detectable markers, it will be apparent that various modifications and improvements can be made to the invention. For example, the deployed bodies may be therapeutic or diagnostic agents in addition to or in lieu of being markers. Moreover, individual features may be shown or otherwise described in one embodiment and not in others, but those skilled in the art will recognize that individual features of one embodiment of the invention can be combined with any or all the features of another embodiment. Accordingly, it is not intended that the invention be limited to the specific embodiments illustrated.

## Claims

1. A magnetic resonance imaging, MRI, imageable marker delivery assembly (10) comprising:
an obturator (12) having an elongated shaft, an internal lumen (18), a proximal end and a substantially sealed distal end (22); and
a marker delivery tube (14) which is configured to be slidably disposed within the internal lumen of the obturator and which has a marker delivery lumen (34) configured to contain one or more tissue markers, a proximal end, and a distal tip, **characterized in that** the distal tip is configured to penetrate the substantially sealed distal end of the obturator to allow passage of the delivery tube therethrough while preventing or minimizing entry of body fluids into the inner lumen of the obturator and has an opening for passage of one or more tissue markers, and
the obturator and the marker delivery tube are formed of a non-magnetic material and the obturator includes an MRI detectable element (78) at a location near the sealed distal end.

2. The marker delivery assembly of claim 1 wherein the marker delivery tube shaft has a plunger (50) which has an elongated shaft with a proximal end and a distal end, which is configured to be slidably disposed within the marker delivery lumen and which is located proximal to the one or more tissue markers.

3. The assembly of claim 1 wherein the substantially sealed distal end of the obturator is a penetratable membrane (28).

4. The assembly of claim 3 wherein the membrane is weakened.

5. The assembly of claim 1 wherein the substantially sealed distal end of the obturator is formed of one or more petals (30).

6. The assembly of claim 1 wherein the substantially sealed distal end of the obturator is a valve (32).

7. The assembly of claim 1 wherein the distal tip of the marker delivery tubular shaft is needle shaped.

8. The assembly of claim 1 wherein the distal tip of the marker delivery tubular shaft is a blunt tip.

9. The assembly of claim 1 wherein the proximal end of the obturator has a hub (60) and the proximal end of the marker delivery tubular shaft has a hub (64) which is configured to fit within the hub of the obturator when the marker delivery shaft is inserted into the elongated shaft of the obturator.

10. The assembly of claim 1 wherein the marker delivery lumen contains a fluid proximal of the at least one tissue markers.

11. The assembly of claim 1 wherein the detectable element is configured to produce a significant image signature.

12. The assembly of claim 11 wherein the detectable element is formed of a material selected from the group consisting of metal, ceramic, metal filled plastic, a radiopaque material, or a mineral filled plastic.

13. The assembly of claim 11 wherein the detectable element is ring shaped.

14. The assembly of claim 11 wherein the detectable element is disposed adjacent to the substantially sealed distal end of the obturator.

15. The assembly of claim 1 wherein a radiopaque material is incorporated into the obturator.

16. The assembly of claim 1 wherein the obturator is formed of a plastic such as polycarbonate.

17. The assembly of claim 1 wherein the elongated shaft of the obturator is filled with air.

18. The assembly of claim 1 including a cannula (24) having an inner lumen configured to receive the obturator.

## Patentansprüche

1. Anordnung (10) zum Ausgeben einer abbildbaren Markierung für die Magnetresonanzbildgebung, MRI, die Folgendes umfasst:
einen Verschluss (12), der einen langgestreckten Schaft, ein internes Lumen (18), ein proximales Ende und ein im Wesentlichen verschlossenes distales Ende (22) besitzt; und
ein Markierungsausgaberohr (14), das konfiguriert ist, in dem internen Lumen des Verschlusses gleitend angeordnet zu werden, und ein Markierungsausgabelumen (34), das konfiguriert ist, ein oder mehrere Gewebemarkierungen zu enthalten, ein proximales Ende und eine distale Spitze besitzt, **dadurch gekennzeichnet, dass** die distale Spitze konfiguriert ist, in das im Wesentlichen verschlossene distale Ende des Verschlusses einzudringen, um den Durchgang des Ausgaberohrs hierdurch zu ermöglichen, während der Eintritt von Körperfluiden in das innere Lumen des Verschlusses verhindert oder minimiert wird, und eine Öffnung für den Durchgang eines oder mehrerer Gewebemarkierungen besitzt, und
der Verschluss und das Markierungsausgaberohr aus einem nicht magnetischen Material gebildet sind und der Verschluss an einer Stelle in der Nähe des verschlossenen distalen Endes ein für die MRI detektierbares Element (78) aufweist.

2. Markierungsausgabeanordnung nach Anspruch 1, wobei der Markierungsausgaberohr-Schaft einen Kolben (50) besitzt, der einen lang gestreckten Schaft mit einem proximalen Ende und einem distalen Ende besitzt und konfiguriert ist, in dem Markierungsausgabelumen gleitend angeordnet zu werden und proximal zu der einen oder den mehreren Gewebemarkierungen angeordnet ist.

3. Anordnung nach Anspruch 1, wobei das im Wesentlichen verschlossene distale Ende des Verschlusses eine durchdringbare Membran (28) ist.

4. Anordnung nach Anspruch 3, wobei die Membran geschwächt ist.

5. Anordnung nach Anspruch 1, wobei das im Wesentlichen verschlossene distale Ende des Verschlusses aus einem oder mehreren Blättern (30) gebildet ist.

6. Anordnung nach Anspruch 1, wobei das im Wesentlichen verschlossene distale Ende des Verschlusses ein Ventil (32) ist.

7. Anordnung nach Anspruch 1, wobei die distale Spitze des Markierungsausgaberohr-Schafts nadelförmig ist.

8. Anordnung nach Anspruch 1, wobei die distale Spitze des Markierungsausgaberohr-Schafts eine stumpfe Spitze ist.

9. Anordnung nach Anspruch 1, wobei das proximale Ende des Verschlusses eine Nabe (60) besitzt und das proximale Ende des Markierungsausgaberohr-Schafts eine Nabe (64) besitzt, die konfiguriert ist, in die Nabe des Verschlusses zu passen, wenn der Markierungsausgabeschaft in den lang gestreckten Schaft des Verschlusses eingesetzt ist.

10. Anordnung nach Anspruch 1, wobei das Markierungsausgabelumen proximal bezüglich der wenigstens einen Gewebemarkierung Fluid enthält.

11. Anordnung nach Anspruch 1, wobei das detektierbare Element konfiguriert ist, eine signifikante Bildsignatur zu erzeugen.

12. Anordnung nach Anspruch 11, wobei das detektierbare Element aus einem Material gebildet ist, das aus der Gruppe gewählt ist, die aus Metall, Keramik, mit Metall gefülltem Kunststoff, einem strahlungsundurchlässigen Material oder einem mit Mineralien gefüllten Kunststoff besteht.

13. Anordnung nach Anspruch 11, wobei das detektierbare Element ringförmig ist.

14. Anordnung nach Anspruch 11, wobei das detektierbare Element in der Nähe des im Wesentlichen verschlossenen distalen Endes des Verschlusses angeordnet ist.

15. Anordnung nach Anspruch 1, wobei ein strahlungsundurchlässiges Material in den Verschluss aufgenommen ist.

16. Anordnung nach Anspruch 1, wobei der Verschluss aus Kunststoff wie etwa Polycarbonat gebildet ist.

17. Anordnung nach Anspruch 1, wobei der lang gestreckte Schaft des Verschlusses mit Luft gefüllt ist.

18. Anordnung nach Anspruch 1, die eine Injektionsnadel (24) enthält, die ein inneres Lumen besitzt, das konfiguriert ist, den Verschluss aufzunehmen.

## Revendications

1. Ensemble de distribution de marqueur imageable par imagerie par résonance magnétique (IRM) (10), comprenant:
un obturateur (12) présentant un arbre allongé, une lumière interne (18), une extrémité proximale et une extrémité distale sensiblement étanche (22); et
un tube de distribution de marqueur (14) qui est configuré de manière à être disposé de façon coulissante à l'intérieur de la lumière interne de l'obturateur et qui comprend une lumière de distribution de marqueur (34) configurée de manière à contenir un ou plusieurs marqueur(s) de tissu, une extrémité proximale et une pointe distale, **caractérisé en ce que** la pointe distale est configurée de manière à pénétrer dans l'extrémité distale sensiblement étanche de l'obturateur afin de permettre le passage du tube de distribution à travers celui-ci tout en empêchant ou en minimisant l'entrée de fluides corporels dans la lumière intérieure de l'obturateur, et comporte une ouverture pour le passage du ou des marqueur(s) de tissu, et l'obturateur et le tube de distribution de marqueur sont constitués d'un matériau non magnétique, et l'obturateur comprend un élément détectable par IRM (78) en un endroit qui est situé à proximité de l'extrémité distale étanche.

2. Ensemble de distribution de marqueur selon la revendication 1, dans lequel l'arbre tubulaire de distribution de marqueur comprend un plongeur (50) qui présente un arbre allongé comprenant une extrémité proximale et une extrémité distale, et qui est configuré de manière à être disposé de façon coulissante à l'intérieur de la lumière de distribution de marqueur et qui est situé à proximité dudit/desdits un ou plusieurs marqueur(s) de tissu.

3. Ensemble selon la revendication 1, dans lequel l'extrémité distale sensiblement étanche de l'obturateur est une membrane pénétrable (28).

4. Ensemble selon la revendication 3, dans lequel la membrane est affaiblie.

5. Ensemble selon la revendication 1, dans lequel l'extrémité distale sensiblement étanche de l'obturateur est constituée d'un ou de plusieurs pétale(s) (30).

6. Ensemble selon la revendication 1, dans lequel l'extrémité distale sensiblement étanche de l'obturateur est une valve (32).

7. Ensemble selon la revendication 1, dans lequel la pointe distale de l'arbre tubulaire de distribution de marqueur est en forme d'aiguille.

8. Ensemble selon la revendication 1, dans lequel la pointe distale de l'arbre tubulaire de distribution de marqueur est une pointe émoussée.

9. Ensemble selon la revendication 1, dans lequel l'extrémité proximale de l'obturateur comprend un moyeu (60), et l'extrémité proximale de l'arbre tubulaire de distribution de marqueur comprend un moyeu (64) qui est configuré de manière à s'agencer à l'intérieur du moyeu de l'obturateur lorsque l'arbre de distribution de marqueur est inséré dans l'arbre allongé de l'obturateur.

10. Ensemble selon la revendication 1, dans lequel la lumière de distribution de marqueur contient un fluide à proximité dudit au moins un marqueur de tissu.

11. Ensemble selon la revendication 1, dans lequel l'élément détectable est configuré de manière à produire une signature d'image significative.

12. Ensemble selon la revendication 11, dans lequel l'élément détectable est constitué d'un matériau qui est sélectionné dans le groupe comprenant un métal, une céramique, un plastique chargé de métal, un matériau radio-opaque ou un plastique à charge minérale.

13. Ensemble selon la revendication 11, dans lequel l'élément détectable est de forme annulaire.

14. Ensemble selon la revendication 11, dans lequel l'élément détectable est disposé à proximité de l'extrémité distale sensiblement étanche de l'obturateur.

15. Ensemble selon la revendication 1, dans lequel un matériau radio-opaque est incorporé dans l'obturateur.

16. Ensemble selon la revendication 1, dans lequel l'obturateur est constitué d'un plastique tel que le polycarbonate.

17. Ensemble selon la revendication 1, dans lequel l'arbre allongé de l'obturateur est rempli d'air.

18. Ensemble selon la revendication 1, comprenant une canule (24) qui présente une lumière intérieure configurée pour recevoir l'obturateur.
